# EUROPÄISCHE PATENTSCHRIFT

(11) **EP 2 061 401 B1**
(45) Veröffentlichungstag und Bekanntmachung des Hinweises auf die Patenterteilung: **27.04.2011**
(21) Anmeldenummer: 07817496.8
(22) Anmeldetag: 07.09.2007
(51) Int. Cl.: A61F 2/38

(54) **KNIEPROTHESE**
KNEE PROSTHESIS
PROTHÈSE DE GENOU

(30) Priorität: 08.09.2006 DE 102006042829
(43) Veröffentlichungstag der Anmeldung: 27.05.2009
(73) Patentinhaber: Siebel, Thomas, 66130 Saarbrücken (DE)
(72) Erfinder: Siebel, Thomas, 66130 Saarbrücken (DE)
(74) Vertreter: Bernhardt, Reinold
(86) Internationale Anmeldenummer: PCT/DE2007/001623
(87) Internationale Veröffentlichungsnummer: WO 2008/028481

(56) Entgegenhaltungen:
- EP-A- 1 518 521
- WO-A-94/09725
- WO-A-2006/092167
- DE-U1-202004 003 133
- US-A- 6 013 103

## Beschreibung

Die Erfindung betrifft eine Knieprothese mit einem Tibiaimplantat, das konkave oder ebene Condylenlaufflächen aufweist, wobei die laterale Condylenlauffläche einen in dorsaler Richtung abfallenden Endabschnitt aufweist.

Eine solche Knieprothese geht aus der US 6,013,103 hervor. In dorsaler Richtung steigt die konkave laterale Conylenlauffläche bis zu einem Abstand von weniger als einem Zehntel ihrer Länge zu ihrem dorsalen Rand an. In dem Endabschnitt zwischen dem dorsalen Rand und dem Bereich bis zu dem die Condylenlauffläche ansteigt, fällt die Condylenlauffläche leicht ab.

Tibiaimplantate werden zusammen mit Femurimplantaten verwendet, welche entsprechend den Laufflächen des Tibiaimplantats einen medialen Condylenteil und einen lateralen Condylenteil umfassen.

Der Erfindung liegt die Aufgabe zugrunde, eine neue Knieprothese der eingangs erwähnten Art zu schaffen, welche in ihrer Funktionsfähigkeit, insbesondere in Bezug auf Knieflexionen, verbessert ist.

Die diese Aufgabe lösende Knieprothese nach der Erfindung ist dadurch gekennzeichnet, dass sich der Endabschnitt in dorsaler Richtung vorzugsweise etwa über ein Fünftel bis ein Drittel der Länge der lateralen Condylenlauffläche erstreckt.

Vorteilhaft ermöglicht der abfallende Endabschnitt der lateralen Condylenlauffläche bei der Knieflexion, die u.a. mit einer Drehung des Femurs um eine zum Tibiaplateau senkrechte Achse verbunden ist, eine problemlose Luxation. Die laterale Femurcondyle bzw. der Femurteil der Knieprothese kann von der lateralen Lauffläche des Tibiaimplantats abgleiten. In herkömmlichen Knieprothesen kommt es bei der Kniebeugung zu einem Anschlag zwischen dem Tibia- und Femurteil der Prothese, was den Beugungswinkel begrenzt.

Die laterale Condylenlauffläche kann über einen konvex gekrümmten Zwischenabschnitt in den abfallenden Endabschnitt übergehen, wobei der Endabschnitt vorzugsweise selbst konvex gekrümmt ist.

In weiterer Ausgestaltung der Erfindung weist der laterale Condylenteil eines Femurimplantats der Knieprothese einen Fortsatz auf, welcher bei der Knieflexion zur Anlage gegen den abfallenden Endabschnitt kommt. Vorteilhaft bildet dieser Fortsatz sowohl einen Hebel als auch eine Führung, der bzw. die das Abgleiten des lateralen Condylenteils von der Condylenlauffläche erleichtert.

Vorzugsweise weist dieser Fortsatz eine zu dem Endabschnitt komplementäre Anlagefläche auf.

In weiterer Ausgestaltung der Erfindung kann die mediale Condylenlauffläche gemäß einer sich in dorsaler Richtung verbreiternden Mulde geformt sein. Vorteilhaft sorgt diese Mulde dafür, dass sich während der Knieflexion der Anlagebereich zwischen der Condylenlauffläche und der medialen Condyle nicht unerwünscht in ventraler Richtung verschiebt.

In einer Ausführungsform der Erfindung ist zwischen den Condylenlaufflächen in der dorsalen Hälfte des Implantats eine Erhöhung mit einer dritten Lauffläche gebildet, auf welcher ein zwischen den Condylenteilen eines Femurimplantats gebildeter Vorsprung abstützbar ist. Vorteilhaft kann durch diese Abstützung bei einer Knieflexion die Rückdrehung des Femurs relativ zum Tibiaimplantat gefördert werden, indem durch die Abstützung die bei der Rückdrehung erforderliche Aufwärtsbewegung des Femurimplantats den abfallenden Endabschnitt hinauf erleichtert wird.

Diese dritte Lauffläche kann teilweise durch einen dorsalen Ansatz am Tibiaimplantat gebildet sein, wobei die dritte Lauffläche vorzugsweise bis zum freien Ende des dorsalen Ansatzes hin ansteigt. Der Drehung des Femurimplantats relativ zum Tibiaimplantat entsprechend verläufe die dritte Lauffläche vorzugsweise im Bogen der Kontur der medialen Condylenlauffläche folgend.

In einer weiteren Ausführungsform der Erfindung geht der abfallende Endabschnitt der lateralen Condylenlauffläche in eine sich senkrecht zum Tibiaplateau erstreckende, weitere Lauffläche über. Vorteilhaft liegt der luxierte Femur nicht gegen den Unterschenkelknochen, sondern gegen das Implantat an.

Bei langandauernden Knieflexionen, z.B. beim Sitzen auf dem Boden im Schneidersitz oder ähnlicher Haltung, werden durch dieses Implantat Belastungen des Unterschenkelknochens vermieden.

Die Erfindung wird nachfolgend anhand von Ausführungsbeispielen und der beiliegenden, sich auf diese Ausführungsbeispiele beziehenden Zeichnungen näher erläutert. Es zeigen:
- Fig. 1: eine Draufsicht auf das Plateau eines Tibiaimplantats nach der Erfin- dung,
- Fig. 2: das Tibiaimplantat von Fig. 1 gemäß Schnittlinie C-C,
- Fig. 3: das Tibiaimplantat von Fig. 1 gemäß Schnittlinie A-A,
- Fig. 4: ein zusammen mit dem Tibiaimplantat von Fig. 1 bis 3 verwendbares Femurimplantat in einer Seitenansicht,
- Fig. 5: das Femurimplantat von Fig. 4 in dorsaler Richtung gesehen,
- Fig. 6 und 7: weitere Ausführungsbeispiele für ein Tibiaimplantat nach der Erfin- dung jeweils in einer Draufsicht auf das Tibiaimplantat,
- Fig. 8: das Tibiaimplantat von Fig. 7 in ventraler Richtung gesehen,
- Fig. 9: ein mit dem Tibiaimplantat von Fig. 7 und 8 in einer Knieprothese ver- wendbares Femurimplantat in ventraler Richtung gesehen, und
- Fig. 10: ein weiteres Ausführungsbeispiel für ein Tibiaimplantat nach der Erfin- dung in einer Seitenansicht in ventraler Richtung,
- Fig. 11: das Tibiaimplantat von Fig. 10 gemäß Schnittlinie B'B, und
- Fig. 12 bis 14: weitere Ausführungsbeispiele für Tibiaimplantate nach der Erfindung.

Ein Tibiaimplantat 1 einer Knieprothese weist eine laterale Condylenlauffläche 2 und eine mediale Condylenlauffläche 3 auf. Die Laufflächen sind im wesentlichen konkav.

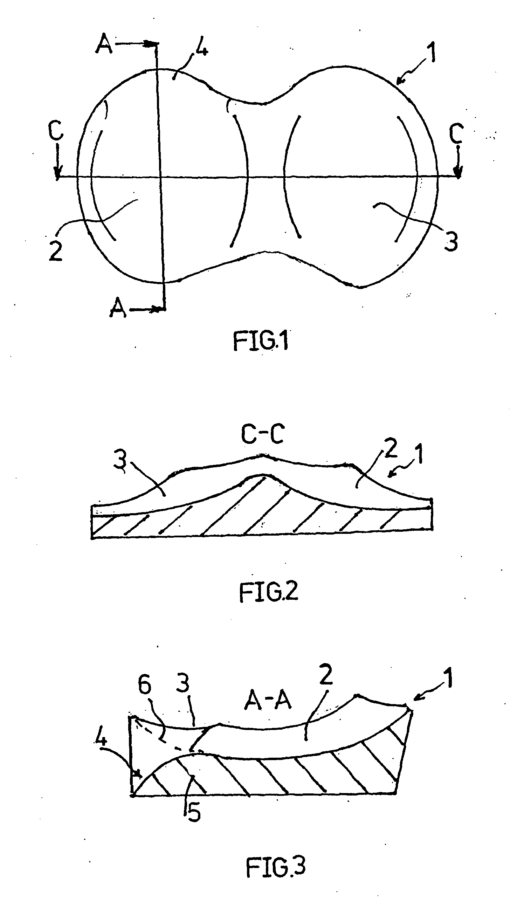

Wie Fig. 3 erkennen lässt, umfasst die laterale Condylenlauffläche 2 einen Endabschnitt 4, welcher in Draufsicht auf das Tibiaplateau, bis zum dorsalen Rand des Implantats hin abfällt. In dem betrachteten Ausführungsbeispiel ist der Endabschnitt 4 konvex gekrümmt. Ebenfalls eine konvexe Krümmung weist ein Übergangsabschnitt 5 zwischen dem konkaven Teil der Condylenlauffläche 2 und dem Endabschnitt 4 auf. Eine Strichlinie 6 deutet den normalen Verlauf der Condylenlauffläche 2 ohne den abfallenden Endabschnitt 4 an.

Bei der Beugung des Knies, bei welcher der Femur in bezug auf eine zum Tibiaplateau senkrechte Achse eine Drehung ausführt, fördert der abfallende Endabschnitt 4 die Luxation und verhindert ein Anschlagen des Femurs bzw. Femurimplantats gegen den dorsalen Rand des Tibiaimplantats 1.

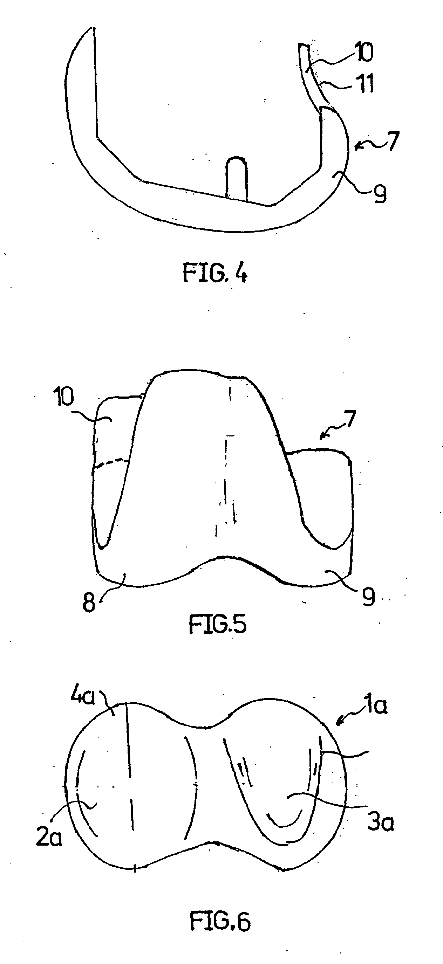

Ein in Fig. 4 gezeigtes Femurimplantat 7 lässt sich vorteilhaft zusammen mit dem anhand der Fig. 1 bis 3 beschriebenen Tibiaimplantat 1 verwenden.

Das Femurimplantat 7 weist einen lateralen Condylenteil 8 und einen medialen Condylenteil 9 auf. An das dorsale Ende des lateralen Condylenteils 8 schließt sich ein Fortsatz 10 mit einer Anlagefläche 11 an, welche etwa komplementär zu dem Endabschnitt 4 der Condylenlauffläche 2 des Tibiaimplantats 1 ausgebildet ist.

Bei einer Knieflexion greift der Fortsatz 10 mit der Anlagefläche 11 an dem Endabschnitt 4 an. Hierdurch ergibt sich einerseits eine Hebelwirkung, andererseits wird durch den anliegenden Fortsatz das Femurimplantat 7 an dem Tibiaimplantat 1 exakt geführt. Beides erleichtert die Luxation, d.h. das Abgleiten des Condylenteils 8 von der Condylenlauffläche 2.

Bei den nachfolgenden Ausführungsbeispielen sind gleiche oder gleichwirkende Teile mit derselben Bezugszahl wie in den vorangehenden Figuren bezeichnet, wobei der betreffenden Bezugszahl der Buchstabe a, b, d, d, e bzw. f beigefügt ist.

Das Tibiaplateau von Fig. 6 unterscheidet sich von dem Tibiaplateau nach Fig. 1 bis 3 dadurch, dass eine mediale Condylenlauffläche 3a muldenartig ausgebildet ist, wobei sich die Breite der Mulde gemäß Konturlinie 12 in dorsaler Richtung verbreitert. Diese Formung der insgesamt konkaven Condylenlauffläche 3a sorgt dafür, dass sich der Anlagebereich der medialen Condyle des Femur bzw. des medialen Condylenteils eines Femurimplantats während der Flexion, bei der sich der Femur um die zum Tibiaplateau senkrechte Achse verdreht, nicht in ventraler Richtung verschiebt, sondern, in ventraler Richtung gesehen, etwa im ersten Drittel der medialen Condylenlauffläche 3a verbleibt.

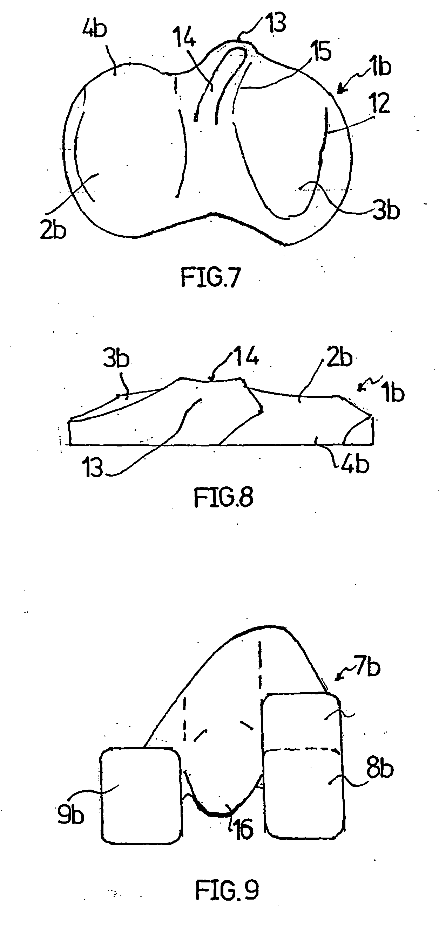

Ein in Fig. 7 gezeigtes Femurimplantat 1 b weist neben einer der Condylenlauffläche 3a entsprechende Condylenlauffläche 3b zusätzlich einen dorsalen Ansatz 13 auf. Durch den Ansatz 13 ist ein Teil einer dritten, sich zwischen den Condylenlaufflächen 2b und 3b erstreckenden Condylenlauffläche 14 gebildet. Diese dritte Lauffläche 14 steigt in dorsaler Richtung bis zum freien Ende des Ansatzes 13 hin an und verläuft bogenförmig etwa einer Kontur 15 der Condylenlauffläche 3b folgend.

Das in Fig. 7 und 8 gezeigte Tibiaimplantat lässt sich in einer Knieprothese zusammen mit einem in Fig. 9 dargestellten Femurimplantat 7b verwenden.

Das Femurimplantat 7b weist neben einem lateralen Condylenteil 8b und einem medialen Condylenteil 9b einen Vorsprung 16 zwischen den Condylenteilen auf.

Soll bei einer Streckung des gebeugten Knies der Femur wieder in die dem ungebeugten Knie entsprechende Drehlage gelangen, so erfordert die damit verbundene Rückdrehung des Femur ein Gleiten der lateralen Condyle 8b, den Endabschnitt 4b hinauf auf den konkaven Teil der Condylenlauffläche 2b. Eine hierbei erforderliche Anhebung des Femur wird dadurch erreicht, dass der Vorsprung 16 während der Streckung zur Anlage gegen die dritte Lauffläche 14 kommt, die entsprechend der erforderlichen Drehung des Femur einen gebogenen Verlauf aufweist.

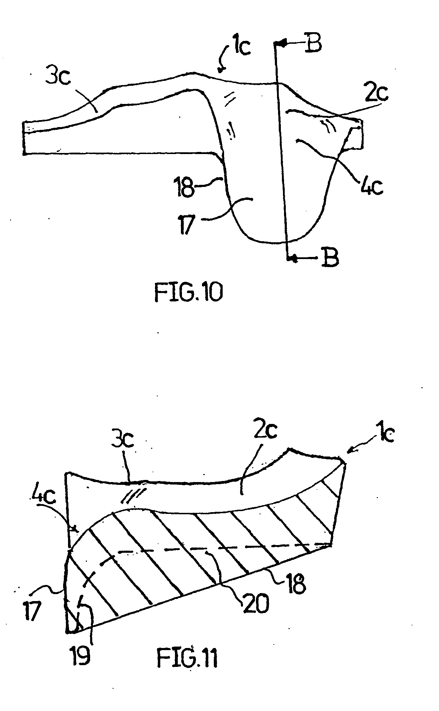

Bei einem in Fig. 10 und 11 gezeigten Tibiaimplantat 1 c geht ein abfallender Abschnitt 4c einer lateralen Condylenlauffläche 2c in eine weitere Lauffläche 17 über, welche sich im wesentlichen senkrecht zum Tibiaplateau erstreckt. Die weitere Lauffläche ist durch einen in Richtung Tibia vorstehenden Ansatz 18 gebildet. In dem gezeigten Ausführungsbeispiel nimmt die vorstehende Länge des Ansatzes 18 bis zum ventralen Rand des Tibiaplateaus 1 c hin linear ab. Eine Ausfräsung, die einen solchen Einsatz aufnimmt, lässt sich während der Implantation mit verhältnismäßig geringem Aufwand herstellen. Alternativ könnte die weitere Lauffläche auch durch eine vorstehende Zunge gebildet sein, wie in Fig. 11 durch eine Strichlinie angedeutet ist.

Bei der Luxation kommt der Femur zur Anlage gegen die zusätzliche Lauffläche 17. Insbesondere bei langandauernden Flexionen, wie sie beim Sitzen auf dem Boden auftreten, schützt das Implantat den Unterschenkelknochen vor Dauerbelastung durch den luxierten Femur.

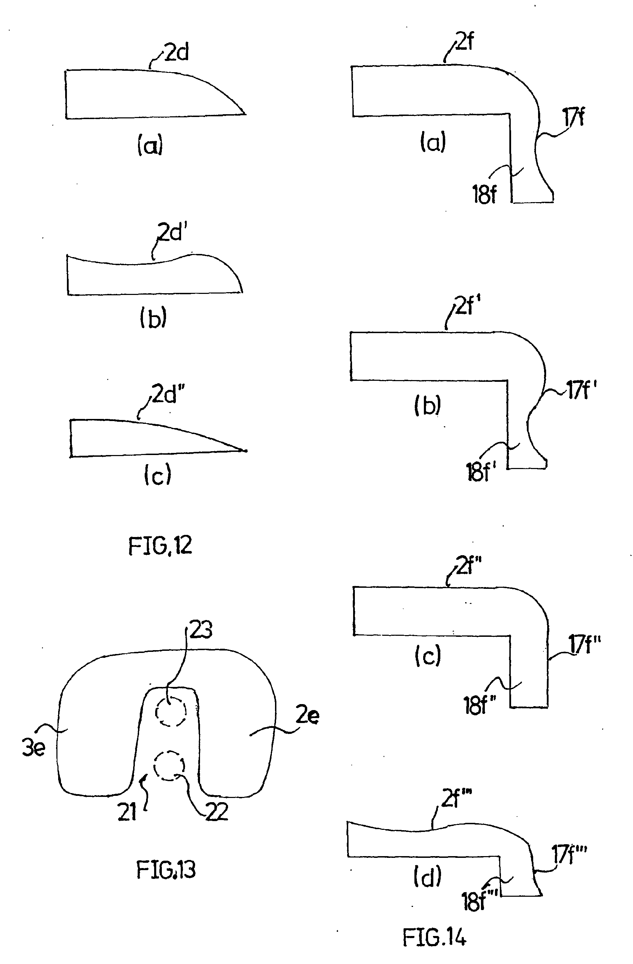

Fig. 12 zeigt weitere Formen von Tibiaimplantanten mit unterschiedlichen Condylenlaufflächen 2d, 2d' und 2d". Die Condylenlauffläche 2d" ist konvex entsprechend einem Kreisbogen geformt.
Fig. 13 zeigt eine Draufsicht auf ein Tibiaimplantant mit Condylenlaufflächen 2e und 3e. Das Implantat weist eine Aussparung 21 auf, durch welche Kreuzbänder 22 und 23 hindurchtreten können.
Fig. 14 zeigt weitere Tibiaimplantate mit Condylenlaufflächen 2f und Ansätzen 18f, wobei die Ansätze unterschiedliche weitere Laufflächen 17f, 17f', 17" und 17'" aufweisen.

## Patentansprüche

1. Knieprothese mit einem Tibiaimplantat (1), das konkave oder ebene Condylenlaufflächen (2,3) aufweist, wobei die laterale Condylenlauffläche (2) einen in dorsaler Richtung abfallenden Endabschnitt (4) aufweist,
**dadurch gekennzeichnet,**
**dass** sich der Endabschnitt (4) in dorsaler Richtung etwa über ein Fünftel bis ein Drittel der Länge der lateralen Condylenlauffläche (2) erstreckt.

2. Knieprothese nach Anspruch 1 ,
**dadurch gekennzeichnet,**
**dass** die laterale Condylenlauffläche (2) über einen konvex gekrümmten Zwischenabschnitt (5) in den abfallenden Endabschnitt (4) übergeht.

3. Knieprothese nach Anspruch 1 oder 2,
**dadurch gekennzeichnet,**
**dass** der Endabschnitt (4) konvex gekrümmt ist.

4. Knieprothese nach einem der Ansprüche 1 bis 3,
**dadurch gekennzeichnet,**
**dass** der laterale Condylenteil eines Femurimplantats (7) der Knieprothese einen Fortsatz (10) aufweist, welcher bei der Knieflexion zur Anlage gegen den Endabschnitt (4) kommt.

5. Knieprothese nach Anspruch 4,
**dadurch gekennzeichnet,**
**dass** der Fortsatz (10) eine zu dem Endabschnitt (4) komplementäre Anlagefläche (11) aufweist.

6. Knieprothese nach einem der Ansprüche 1 bis 5,
**dadurch gekennzeichnet,**
**dass** die mediale Condylenlauffläche (3a;3b) eines sich in dorsaler Richtung verbreiternde Mulde (12) aufweist.

7. Knieprothese nach einem der Ansprüche 1 bis 6,
**dadurch gekennzeichnet,**
**dass** zwischen den Condylenlaufflächen (2b,3b) in der dorsalen Hälfte des Implantats eine Erhöhung mit einer dritten Lauffläche (14) gebildet ist, auf welcher sich ein zwischen den Condylenteilen (8b,9b) eines Femurimplantats (7b) gebildeter Vorsprung (16) abstützen lässt.

8. Knieprothese nach Anspruch 7,
**dadurch gekennzeichnet,**
**dass** die dritte Lauffläche (14) teilweise durch einen dorsalen Ansatz (13) an dem Tibiaimplantat (1b) gebildet ist.

9. Knieprothese nach Anspruch 8,
**dadurch gekennzeichnet,**
**dass** die dritte Lauffläche (14) bis zum freien Ende des dorsalen Ansatzes (13) hin ansteigt.

10. Knieprothese nach Anspruch 8 oder 9,
**dadurch gekennzeichnet,**
**dass** die dritte Lauffläche (14) im Bogen, etwa der Kontur (15) der medialen Condylenlauffläche (3b) folgend, verläuft.

11. Knieprothese nach einem der Ansprüche 1 bis 10,
**dadurch gekennzeichnet,**
**dass** der abfallende Endabschnitt (4c) der lateralen Condylenlauffläche (2c) in eine sich senkrecht oder im Mittel senkrecht zum Tibiaplateau erstreckende, dorsale Lauffläche (17) übergeht.

12. Knieprothese nach Anspruch 11,
**dadurch gekennzeichnet,**
**dass** die weitere Lauffläche (17) durch einen Ansatz (18) gebildet ist, welcher von den in Laufflächen (2c,3c) abgewandten Seite des Tibiaimplantats (1 c) vorsteht.

13. Knieprothese nach Anspruch 12,
**dadurch gekennzeichnet,**
**dass** die vorstehende Länge des Ansatzes (18) zur ventralen Seite des Tibiaimplantats (1c) hin abnimmt, vorzugsweise linear abnimmt.

## Claims

1. Knee prosthesis with a tibial implant (1) that has concave or plane condylar bearing surfaces (2, 3), wherein the lateral condylar bearing surface (2) has an end portion (4) that slopes downward in the dorsal direction, **characterized in that** the end portion (4) extends in the dorsal direction along approximately one fifth to one third of the length of the lateral condylar bearing surface (2).

2. Knee prosthesis according to Claim 1, **characterized in that** the lateral condylar bearing surface (2) merges into the downwardly sloping end portion (4) via a convexly curved intermediate portion (5).

3. Knee prosthesis according to Claim 1 or 2, **characterized in that** the end portion (4) is convexly curved.

4. Knee prosthesis according to one of Claims 1 to 3, **characterized in that** the lateral condylar part of a femoral implant (7) of the knee prosthesis has a continuation (10) that comes to rest against the end portion (4) when the knee is flexed.

5. Knee prosthesis according to Claim 4, **characterized in that** the continuation (10) has a contact surface (11) complementary to the end portion (4).

6. Knee prosthesis according to one of Claims 1 to 5, **characterized in that** the medial condylar bearing surface (3a; 3b) has a depression (12) that widens in the dorsal direction.

7. Knee prosthesis according to one of Claims 1 to 6, **characterized in that**, between the condylar bearing surfaces (2b, 3b), in the dorsal half of the implant, an elevation with a third bearing surface (14) is formed on which a projection (16) formed between the condylar parts (8b, 9b) of a femoral implant (7b) can be supported.

8. Knee prosthesis according to Claim 7, **characterized in that** the third bearing surface (14) is formed partially by a dorsal extension (13) on the tibial implant (1b).

9. Knee prosthesis according to Claim 8, **characterized in that** the third bearing surface (14) slopes upward as far as the free end of the dorsal extension (13).

10. Knee prosthesis according to Claim 8 or 9, **characterized in that** the third bearing surface (14) extends in an arch that approximately follows the contour (15) of the medial condylar bearing surface (3b).

11. Knee prosthesis according to one of claims 1 to 10, **characterized in that** the downwardly sloping end portion (4c) of the lateral condylar bearing surface (2c) merges into a dorsal bearing surface (17) that extends perpendicular to or approximately perpendicular to the tibial plateau.

12. Knee prosthesis according to Claim 11, **characterized in that** the further bearing surface (17) is formed by an extension (18) that protrudes from the side of the tibial implant (1c) directed away from the bearing surfaces (2c, 3c).

13. Knee prosthesis according to Claim 12, **characterized in that** the protruding length of the extension (18) decreases, preferably linearly, toward the ventral side of the tibial implant (1c).

## Revendications

1. Prothèse du genou comprenant un implant tibial (1) qui comporte des surfaces de réception de condyle (2, 3) concaves ou planes, la surface de réception de condyle latéral (2) comprenant un tronçon terminal (4) qui descend en direction dorsale,
**caractérisée en ce que**
le tronçon terminal (4) s'étend en direction dorsale approximativement sur un cinquième à un tiers de la longueur de la surface de réception de condyle (2) latéral.

2. Prothèse du genou selon la revendication 1,
**caractérisée en ce que** la surface de réception de condyle latéral (2) se transforme dans le tronçon terminal (4) descendant via un tronçon intermédiaire à courbure convexe (5).

3. Prothèse du genou selon la revendication 1 ou 2,
**caractérisée en ce que** le tronçon terminal (4) a une courbure convexe.

4. Prothèse du genou selon l'une des revendications 1 à 3,
**caractérisée en ce que** la partie de condyle latéral d'un implant fémoral (7) de la prothèse de genou comporte un prolongement (10) qui, lors de la flexion du genou, vient en contact contre le tronçon terminal (4).

5. Prothèse du genou selon la revendication 4,
**caractérisée en ce que** le prolongement (10) comporte une surface d'appui (11) complémentaire au tronçon terminal (4).

6. Prothèse du genou selon l'une des revendications 1 à 5,
**caractérisée en ce que** la surface de réception de condyle médial (3a ; 3b) comporte une dépression (12) qui s'élargit en direction dorsale.

7. Prothèse du genou selon l'une des revendications 1 à 6,
**caractérisée en ce que**, entre les surfaces de réception de condyle (2b, 3b) dans la moitié dorsale de l'implant, il est formé une surélévation avec une troisième surface de réception (14) contre laquelle une saillie (16) formée entre les parties de condyle (8b, 9b) d'un implant fémoral (7b) peut venir s'appuyer.

8. Prothèse du genou selon la revendication 7,
**caractérisée en ce que** la troisième surface de réception (14) est formée au moins partiellement par un talon dorsal (13) sur l'implant tibial (1b).

9. Prothèse du genou selon la revendication 8,
**caractérisée en ce que** la troisième surface de réception (14) monte jusqu'à l'extrémité libre du talon dorsal (13).

10. Prothèse du genou selon la revendication 8 ou 9,
**caractérisée en ce que** la troisième surface de réception (14) s'étend sur un arc, en suivant approximativement le contour (15) de la surface de réception de condyle médial (3b).

11. Prothèse du genou selon l'une des revendications 1 à 10, **caractérisée en ce que** le tronçon terminal descendant (4c) de la surface de réception de condyle latérale (2c) se transforme en une surface de réception dorsale s'étendant perpendiculairement ou perpendiculairement au milieu par rapport au plateau tibial.

12. Prothèse du genou selon la revendication 11,
**caractérisée en ce que** l'autre surface de réception (17) est formée par un talon (18) qui fait saillie du côté de l'implant tibial (1c) détourné des surfaces de réception (2c, 3c).

13. Prothèse du genou selon la revendication 12,
**caractérisée en ce que** la longueur en saillie du talon (18) diminue, de préférence de façon linéaire, en direction du côté ventral de l'implant tibial (1c).
